# EUROPEAN PATENT SPECIFICATION

(11) **EP 4 531 690 B1**
(45) Date of publication and mention of the grant of the patent: **18.02.2026**
(21) Application number: 23727584.7
(22) Date of filing: 22.05.2023
(51) Int. Cl.: A61B 6/04, A61B 6/10, A61G 10/00

(54) **DIAGNOSTIC CONTAINER FOR OPTIMIZED MEDICAL IMAGING**
DIAGNOSEBEHÄLTER ZUR OPTIMIERTEN MEDIZINISCHEN BILDGEBUNG
RÉCIPIENT DE DIAGNOSTIC POUR IMAGERIE MÉDICALE OPTIMISÉE

(30) Priority: 31.05.2022 EP 22176320
(43) Date of publication of application: 09.04.2025
(73) Proprietor: Koninklijke Philips N.V., 5656 AG Eindhoven (NL)
(72) Inventor: LEUSSLER, Christoph Günther, 5656 AG Eindhoven (NL); VOGTMEIER, Gereon, 5656 AG Eindhoven (NL); WEISS, Steffen, 5656 AG Eindhoven (NL); CHAKRABARTI, Biswaroop, 5656 AG Eindhoven (NL)
(74) Representative: Philips Intellectual Property & Standards
(86) International application number: PCT/EP2023/063557
(87) International publication number: WO 2023/232510

(56) References cited:
- WO-A1-2021/212118
- WO-A2-2008/079429
- US-A1- 2022 040 020
- US-A1- 2022 160 564

## Description

### FIELD OF THE INVENTION

The present invention relates to a diagnostic container for hermetically enclosing a patient during imaging with a medical imaging system, and to the use of such a container in a medical imaging system.

### BACKGROUND OF THE INVENTION

In a hospital environment, there is a need to safely transport patients between different clinical departments like imaging and cancer treatment centers. Patients with cancer treatment and burned victims are sensitive to airborne pathogens and on the other side infected patients need to be safely transported without the risk of infecting staff or other patients. With the advent of wide bore magnetic resonance imaging (MRI) scanners with a diameter of the bore of even more that 80 cm, patients can be imaged even when they are located in a diagnostic shell. In addition, MRI system with low field strength of about 0.6 Tesla appear on the market which reduced demands on acoustic noise protection. Similar options are available for computed tomography (CT) scans using a large bore CT scanner. Diagnostic scanners that are used for imaging of highly infectious patients in a diagnostic shell do not have to be disinfected after an imaging session. Workflows can be optimized regarding time and cost and it can be ensured that diagnostic scanners can remain in operation without long service times.

The transport in a diagnostic sphere allows the diagnostic operator and staff not to wear full protective wear and equipment to protect from an infection. The diagnostic sphere can be configured to either protect the surroundings from the patient, or to protect the patient from the surroundings. There is no need to disinfect the MRI scanner, the radiofrequency (RF) equipment or the patient preparation room. The diagnostic sphere helps for safe diagnostic imaging and transportation of infectious patients infected with diseases like Ebola, SARS, gastroenteritis outbreaks like norovirus, MERS and COVID. In addition, the confined and protected environment may help to keep the patient away from frightening technical equipment and can generate a virtual environment by implementing a virtual reality. Various transport solutions have been developed to transport infectious patients with single use or multi use versions of a diagnostic shell.

Besides transportation of infectious patient in a hospital environment, a further issue is diagnostic imaging within an MRI, CT or an X-ray system. For MRI local coils need to be positioned close to the patient, while they need to be removed for CT and X-ray imaging.

WO 2021/212118 A1 discloses a patient isolation unit for use with a medical imaging system including an enclosure comprised of a pathogen impermeable material.

The inventors of the present invention have thus found that it would be advantageous to have an improved diagnostic container for transport and imaging of infectious patients that allows easy positioning of medical equipment like RF coils of a magnetic resonance imaging system close to the patient.

### SUMMARY OF THE INVENTION

It is an object of the present invention to provide a diagnostic container for hermetically enclosing a patient during imaging with a medical imaging system that allows safe and optimized imaging of a patient while maintaining an airtight enclosure of the patient.

The object of the present invention is solved by the subject matter of the independent claims, wherein further embodiments are incorporated in the dependent claims.

The described embodiments similarly pertain to the diagnostic container for hermetically enclosing a patient during imaging with a medical imaging system, and to the use of such a container in a medical imaging system. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first embodiment of the invention, there is provided a diagnostic container for hermetically enclosing a patient during imaging with a medical imaging system. The container comprises an airtight shell configured for providing an airtight enclosure of the patient, wherein the airtight shell comprises at least one recess extending into an inner volume of the airtight shell. The recess is configured for ensuring the airtight enclosure, and the recess is further configured for receiving a component part of a medical device and for allowing a positioning of the component part in close proximity to the patient without direct contact of the component part to the patient.

Thus, the container and the airtight shell are configured to encompass the patient and provide a protective casing that prevents free circulation of air and pathogens between the inside and the outside of the container. The container is configured to be arranged inside of the bore of a medical imaging system with the patient being in the container. The container is provided with at least one dedicated recess that can be formed as a connected opening, which allows positioning of, for example, an RF coil like a body coil or a sensor without direct contact to the patient. The medical device to be located in the recess can be an active or passive device, like, for example, an MRI coil, a device for vital sensing, a therapy device, or radiation protection. The recess extends into the inner volume of the diagnostic container, and thus allows the positioning of the coil or the sensor close to the patient inside of the container. However, as the recess can be part of the airtight shell, the recess also prevents circulation of air and pathogens between the inside and the outside of the container. The recess can be formed like a pocket with airtight and closed walls on each side except the entrance side. Alternatively, the recess can be formed like a channel or a tunnel reaching through the entire inner volume of the diagnostic container.

Therefore, for example, a body coil, a sensor, or another medical treatment device can be flexibly located close to the patient from the outside of the airtight shell, such that the coil, the sensor or the device extends into the inner volume of the container without braking the airtight enclosure. This is ensured, as an airtight wall of the recess remains between the device inserted into the recess and the patient. The airtight shell or the airtight enclosure can be configured to have more than one recess. For example, an MRI coil can be positioned in a first recess, while a vital signs monitor can be positioned in a second recess.

Thus, an MRI compatible diagnostic container according to the invention allows safe positioning of RF coils and sensors on or close to the patient without having direct contact to the patient, and without allowing pathogens polluting the coils or the sensor. The opening or the recess can be configured as inserting windows with different length and width adapted to the dedicated coils to be applied by the recess to the patient. A container can comprise a plurality of recesses at different locations of the container and/or with different size. The recess can be configured to be opened and closed using a zipper or a shutter.

The components installed in the diagnostic container like breathing support, monitoring, etc. are preferably built and installed in a way that imaging is not disturbed. For example, these components can be installed outside of the field of view of the medical imaging system.

The diagnostic container according to the invention can overcome the drawbacks of a low imaging quality of systems with integrated body coil, a sit allows dedicated RF coils to be located on or close to the patient even in combination with an airtight enclosure. As the coil has no direct contact to the patient, the protection against infection is upheld. The transportation container can be compatible for different imaging systems like CT, X-ray or MRI, and disinfection of the diagnostic imaging system after each patient is not necessary any more. The airtight shell providing the enclosure can be made of a soft material, but can also be combined with and/or supported by a mechanical or pneumatic modular holder, so that the enclosure can be formed and allows to support sensors and RF coils located above the patient. In addition, communication and monitoring between patient and a local or remote operator can be preferably performed via the diagnostic container.

In an embodiment of the invention, the container comprises a patient bed configured for receiving the patient, and the patient bed is arranged inside of the airtight shell.

Thus, the patient bed to receive the patient can be arrange inside the volume confined by the diagnostic container. One recess can be located above the patient bed and the patient, and one recess can be located below the patient, for example. In this embodiment, the container is provided with a patient bed and connected openings to allow free positioning of RF coils and therapy devices for ablation or hyperthermia, for example. One recess can be arranged to position a medical device and/or a sensor inside of the patient bed, like a thin vital signs sensor mattress, an MRI coil, an antenna sheet, or a radar sensor.

In an embodiment of the invention, the container comprises a patient bed, wherein the airtight shell is configured to be connected to the patient bed, and the airtight enclosure of the patient is provided by the airtight shell in combination with the patient bed.

In this example, the patient can be laid onto the patient bed, and the airtight shell encompasses the patient and is connected to the patient bed in order to provide the protective enclosure. The container can be positioned on the patient bed and connected via seals and spring fittings for tight mechanical contact. The container does may have recesses on both sides of the container which can be mechanically interconnected via a flexible material to allow positioning of planar flexible coils on the patient.

Alternatively, the container can be constructed such, that it fits with the MRI patient bed. In this embodiment, the patient can be arranged inside of the diagnostic container, and the container together with the patient inside can be placed onto a standard MRI bed.

In an embodiment of the invention, the airtight shell comprises an access port, wherein the access port comprises an opening and a seal, wherein the access port is configured to allow insertion of a head coil of the medical imaging system through the opening to position the head coil in close proximity to the head of the patient, and wherein the seal is configured to ensure the airtight enclosure.

Thus, the container can be configured such that a head coil of an MRI system can be shifted via seals onto the patient. Therefore, the container can comprise an opening adjacent to the head of the patient, and seals are provided around the opening, such that in combination with the head coil inserted through the opening, the airtight enclosure is assured. This configuration allows preferably a linear movement of the coil in the direction towards the patient to adjust an optimal distance to the head of the patient. The distance of the head coil from the head of the patient is preferably in the range of 5 mm to 10 cm.

The medical imaging system is a magnetic resonance imaging system and the component part of the medical device is a body coil of the magnetic resonance imaging system.

The container can be configured to be compatible with a magnetic resonance imaging system and with an X-ray or CT system, such that the patient can be imaged in both systems consecutively without the necessity to change or open the container. Thus, a patient can be scanned with X-ray and in a second session RF coils can be applied to prepare the container for an MRI examination. The container can also be compatible with an MRI and a therapy system such as a hyperthermia system. The recess can be used for applying RF MRI coils but also or simultaneously applicators for hyperthermia.

In an embodiment of the invention, the container comprises an interface configured to connect the container to the medical imaging system to provide electric power supply, ventilation and/or data transfer between the container and the medical imaging system.

This interface between diagnostic scanner and container can provide AC or DC supply, the identification of the container, the supply of the RF coils with the RF signal, and/or communication regarding the monitoring of vital signs of the patient. The diagnostic system can be loaded with a configuration file to be compatible with respect to identification, communication and system tuning.

The interface connection to the MRI system for ventilation, electronic power supply and/or communication can be provided via an integrated connector. The electronic monitoring and management system of the diagnostic container can be connected to the diagnostic scanner via an interface.

There may be a need for the individual diagnostic containers to be controlled, distributed and collected by a software management system. Individual diagnostic containers may need to be correctly labelled, positioned, disinfected, stored, etc. Detection of position in the hospital environment may be realized using an ultra-wideband radar (UWB) or a 5G telecommunication network, preferably controlled by a health suite like a cloud-based shared software program. Further acoustic noise reduction may be realized by foam pad between the diagnostic container and the patient bed. The diagnostic container may preferably apply passive acoustic noise reduction. The active noise cancelling system may use distributed nonmagnetic piezo sensing for acoustic and mechanical vibration. Additionally, headphones may be applied via the recess or head phones are may be applied before the patient is located in the diagnostic container.

In an embodiment of the invention, the airtight shell is a mechanical hardcover or a sliding tent roof.

The airtight shell as the cowling can be constructed as a mechanical hard cover or as a sliding tent roof that preferably is adapted to the specifications of the imaging modality. Opening and closing of the diagnostic container may be realized by a fully automated control system, so that infection risk can be controlled. The output of different sensors (like the open or close status of the container, the status of the MRI or X-Ray system, or compatibility with the diagnostic system, etc.) can be fed to a trained neuronal network, which can be configured to control the infection risk by clear monitoring and an alarm signal to the operator. The output of the trained network can also be a risk assessment level indicator. In addition, a guidance and/or an indication to the staff can be provided, what needs to be modified or added in order to reduce the risk level. The trained network hardware and software can be located in a module at the diagnostic container.

In an embodiment of the invention, the interface is arranged at and end of the container corresponding with the head or with the feet of the patient.

Electronic communication electronics like the interface is preferably located at the front or back side of the container to prevent imaging artifacts. Distributed sensing electronics inside of the diagnostic container may be realized via optical or wireless communication. For wired communication, high impedance RF filtering may be necessary. A connectable ventilation system to the diagnostic scanner allows to provide air with over pressure or under pressure. The ventilation can be realized by a dockable ventilation system with a sleeve and/or plug and play to realize sufficient distance from locations that are incompatible with the medical imaging system.

In an embodiment of the invention, the container comprises an optical window configured for camera-based monitoring of a vital sign of the patient through the optical window.

Vital signs such as breathing and heartbeat can be monitored by a camera, which is particularly advantageous for infectious patients. Breathing can be monitored via motion detection and heartbeat can be detected by remote photo-plethysmography of the face, for example. An optical compatible window is therefore added to the diagnostic container to allow vital signs monitoring of the patient via an external camera. The window is preferably located and oriented such that minimal reflections are realized. An integrated mirror can further provide an optical path to the surface of the patient.

In an embodiment of the invention, the container comprises a feedthrough for an optical fiber configured to be attached to the body of the patient via a skin patch.

For single use diagnostic containers comprising transparent and in-transparent foils, breathing motion detection may be possible, but remote photo-plethysmography of the face may be difficult through a foil, because any tiny motion of the foil may possibly corrupt the signal. Therefore, a single use optical adhesive patch can be integrated into the single use diagnostic container, which is internally attached to the patient's temple or neck, for example. An optical window of fiber-optic access for camera-based vital signs monitoring can be added to the container. A plastic optical fiber that is cheap for single use can be used to both illuminate the skin and guide the reflected light through the feedthrough of the container to the outside. On the outside, the fiber can be fitted with a plug that fits to the camera housing of an imaging system. The camera then can see the colour change of the skin in some peripheral area of its field of view. For illumination, a further fiber may be plugged into the light source, if it is separated from the camera.

In an embodiment of the invention, the airtight shell is made of a material with low X-ray absorption and/or low absorption for radiation for oncology therapy treatment. In addition or alternatively, the airtight shell can be made of a material that is essentially transparent for the radiation to be detected in PET imaging.

A diagnostic container compatible with X-ray and CT preferably has X-ray transparent materials for the housing with low absorption for X-rays, and preferably all elements that are in the field of view of the diagnostic scanner comprise low X-ray absorbing materials. In addition or alternatively, the container can comprise an X-ray transparent window for dedicated areas in the field of view of the scanner. Thus, dedicated areas of the diagnostic container can be made out of low X-ray absorbing and/or low scattering material to allow for good three-dimensional CT image quality using the transmission path at the dedicated patient position. Further, the container can be made at least partially from a material that comprises low absorption for radiation that can be used for oncology therapy treatment, such as radiation from linear accelerators or synchrotrons like proton radiation.

In an embodiment of the invention, the container comprises a marker configured to provide an indication of a position of the patient with respect to the medical imaging system. Thus, local markers can be located on the container for improved positioning of the patient inside the diagnostic scanner and/or for improved positioning of the container inside of the medical imaging system.

In an embodiment of the invention, the airtight shell comprises a plurality of modules, wherein the modules are configured to be connected to each other in order to provide the airtight enclosure and/or wherein the modules comprise a communication link to be connected communicationally to each other.

Thus, the container can be divided into a plurality of modules that can be connected to each other in order to provide the airtight enclosure. In addition or alternatively, each module can comprise a communication link to connect the module to the adjacent module. As for medical imaging or radiation therapy, the container needs to be transparent for the respective radiation, the material of the container is preferably transparent. A modular solution allows to connect and insert a module comprising a window that is transparent for X-ray radiation or therapeutic radiation at the respective position. A communication link between the individual modules for wireless or optical communication can be configured to transmit signals to control the container or to provide comfort to the patient. Thus, the link can control airtightness, the mechanical connection via a fastener, loom, or guidance for assembly.

In an embodiment of the invention, the container comprises an airlock with a membrane, wherein the airlock is configured to allow contact imaging of the body of the patient through the membrane.

This access port can be configured to allow contact imaging like ultrasound, for example, as imaging modalities like ultrasound require contact with the patient. An airlock mechanism allows performing such imaging procedures while maintaining asepsis and thus ensuring the airtight enclosure. During operation, the port can be fitted with a membrane like a sock. This membrane can be made from latex or silicone or another appropriate material, and can optionally be covered with an impedance matching gel, and thus be similar in principle to an isolation glove box. Thus, through the access port, the ultrasound imaging can be performed while avoiding actual physical contact. The membrane can be sterilizable or disposable. In the latter case, there can be an airlock cover on the inner side of the port inside of the membrane or on both sides. The membrane can be aseptically replaced after closing the airlocks.

According to another aspect of the invention, there is provided the use of a container according to any of the preceding embodiments in a medical imaging system.

This method of using the container in a medical imaging system can comprise the steps of providing a container according to any of the preceding embodiments, placing a patient inside of the container and closing the container hermetically, arranging the container in a medical imaging system, arranging a component part like a body coil of a magnetic resonance imaging system in the recess of the container, and imaging the patient with the medical imaging system.

Thus, the benefits provided by any of the above aspects equally apply to all of the other aspects and vice versa.

In a gist, the invention relates to a diagnostic container for hermetically enclosing a patient during imaging with a medical imaging system. The container comprises an airtight shell configured for providing an airtight enclosure of the patient, and the airtight shell comprises at least one recess extending into an inner volume of the airtight shell, wherein the recess is configured for ensuring the airtight enclosure. The recess is further configured for receiving a component part of a medical device and for allowing a positioning of the component part in close proximity to the patient without direct contact of the component part to the patient.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic set-up of a diagnostic container for hermetically enclosing a patient during imaging with a medical imaging system according to an embodiment of the invention.
Fig. 2 shows a schematic set-up of a diagnostic container for hermetically enclosing a patient during imaging with a medical imaging system according to another embodiment of the invention.
Fig. 3 shows a schematic set-up of a diagnostic container for hermetically enclosing a patient during imaging with a medical imaging system according to another embodiment of the invention.
Fig. 4 shows a schematic set-up of a diagnostic container for hermetically enclosing a patient during imaging with a medical imaging system according to another embodiment of the invention.
Fig. 5 shows a schematic set-up of a diagnostic container for hermetically enclosing a patient during imaging with a medical imaging system including a device for camera-based monitoring of the patient according to another embodiment of the invention.
Fig. 6 shows a block diagram of a method of using a container according to an embodiment of the invention in a medical imaging procedure.
Fig. 7A shows a schematic set-up of an interface connection between a medical imaging system and a diagnostic container according to an embodiment of the invention.
Fig. 7B shows a flowchart of a software protocol between a medical imaging system and a diagnostic container according to an embodiment of the invention.

### DETAILED DESCRIPTION OF EMBODIMENTS

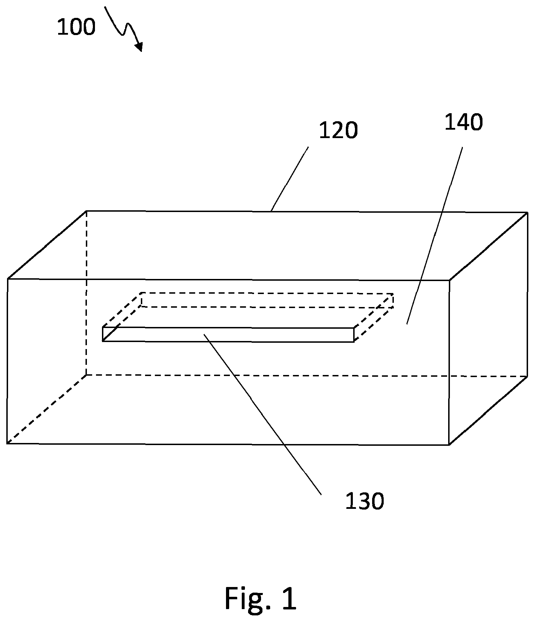

Fig. 1 shows a schematic set-up of a diagnostic container 100 for hermetically enclosing a patient 110 during imaging with a medical imaging system according to an embodiment of the invention. The container comprises an airtight shell 120 surrounding an inner volume 140 of the container. A recess 130 is provided in the airtight shell 120 of the container 100 extending into the inner volume 140 of the container 100. The space inside of the recess 130 is divided from the inner volume 140 of the container 100 by the walls of the recess thereby enduring the airtight enclosure. A component part 150 of a medical system like a body coil, a sensor, or a treatment device can be inserted into the recess 130 and thus can be positioned close to a patient 110 inside of the container, without the risk of pathogens being transferred from the patient 110 to the component part 150 and vice versa, and without the risk of pathogens leaving or entering the inner volume 140 of the container 100.

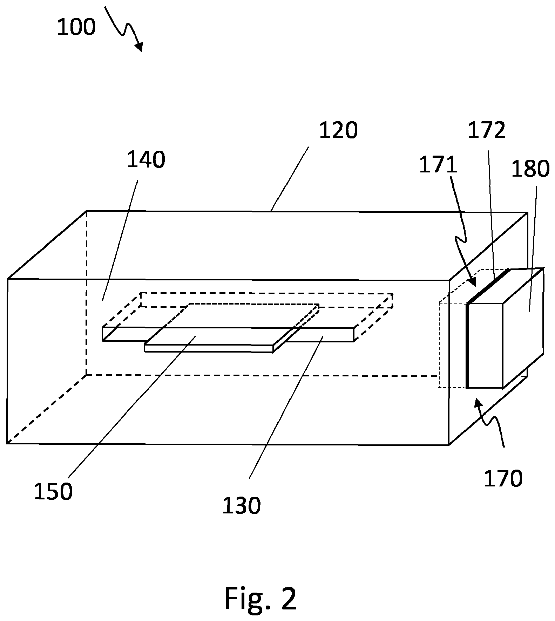

Fig. 2 shows a schematic set-up of a diagnostic container 100 for hermetically enclosing a patient 110 during imaging with a medical imaging system according to another embodiment of the invention. In this figure, a component part 150 of a medical imaging system like a body coil is inserted into the recess 130 of the container 100. In addition, the container is provided with an access port 170. The access port comprises an opening 171 in the airtight shell 120 of the container 100, and a seal 172 surrounding the opening. When a device like a head coil 180 of the medical imaging system is inserted into the opening 171 of the access port 170, the seal 172 gets into contact with the head coil and seals the inner volume of the container against the outside to ensure the airtight enclosure of the container 100.

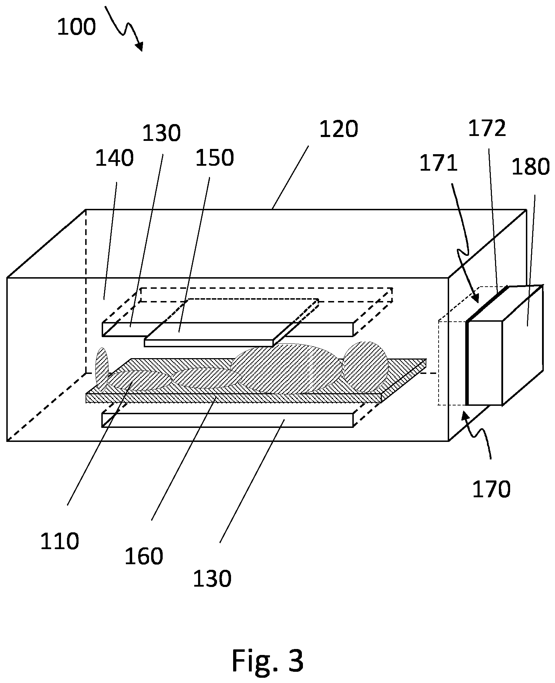

Fig. 3 shows a schematic set-up of a diagnostic container 100 for hermetically enclosing a patient 110 during imaging with a medical imaging system according to another embodiment of the invention. In this figure, the container comprises a second recess, and a patient 110 is lying on a patient bed 160 in between of the two recesses 130. A component part 150 of the imaging system can be inserted in one or both recesses 130. In addition, in this embodiment, the container comprises an access port adjacent to the head of the patient 110 in order to receive a head coil 180 of the imaging system to be located close to the head of the patient 110.

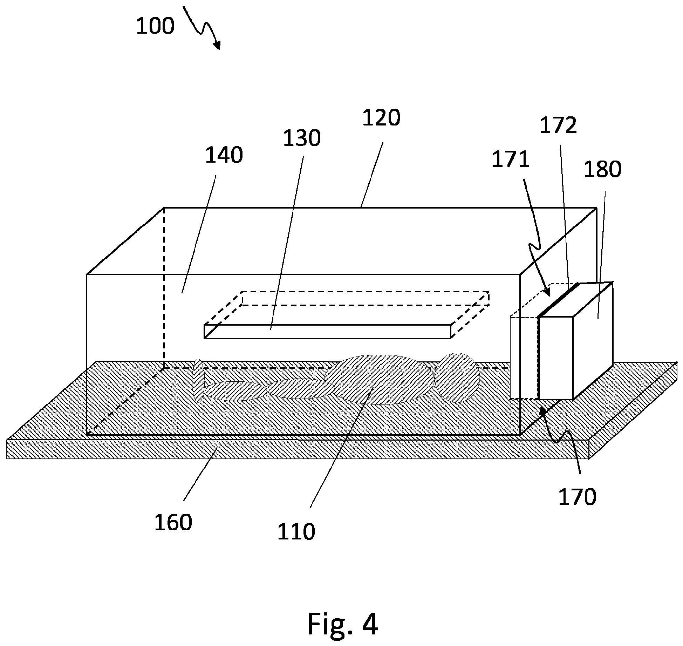

Fig. 4 shows a schematic set-up of a diagnostic container 100 for hermetically enclosing a patient 110 during imaging with a medical imaging system according to another embodiment of the invention. In this embodiment, the airtight shell 120 of the diagnostic container 100 is connected to the patient bed 160, such that the patient bed 160 and the airtight shell 120 in combination provide the airtight enclosure to prevent pathogens from spreading. The patient 110 is located inside of the container 100. A body coil can be inserted into the recess 130 to be located close to the patient 110. The MRI patient bed can be provided with seals to allow adaptation of the container for hermetically enclosing the patient. Alternatively, the container 100 including the airtight shell 120 can be arranged on a patient bed 160. The container 100 can be provided with a recess 130 and a sealed opening to allow positioning of RF coils and sensors.

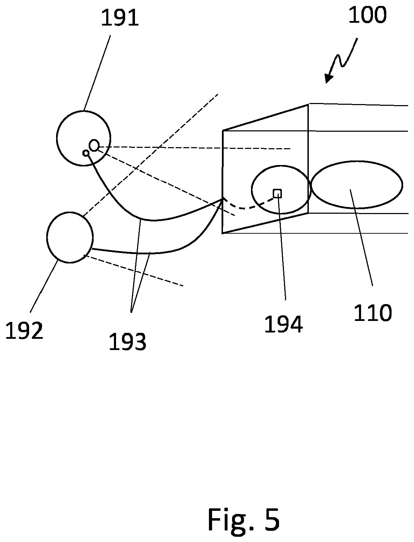

Fig. 5 shows a schematic set-up of a diagnostic container 100 for hermetically enclosing a patient 110 during imaging with a medical imaging system including a device for camera-based monitoring of the patient according to another embodiment of the invention. In this embodiment shown in Fig. 5, the diagnostic container 100 is provided with a feedthrough for an optical fiber 193. The optical fiber is connected on the one side to a skin patch 194 attached to the skin of a patient 110 inside of the container. Via a beam splitter, the other side of the optical fiber 193 can be connected to a light source 192 and a camera 191, and the light of the light source is transmitted via the optical fiber 193 to the skin patch 194, and the camera 191 receives the light backscattered from the skin of the patient 110. Thus, the container 100 can be provided with an optical fiber connection to guide a photo plethysmography signal from a skin patch 194 directly into the peripheral field of view of the camera 191, which can also be used for monitoring of vital signs via movement of the patient like breathing motion. A second fiber may be connect to the light source 192 if this is separated from the camera 191.

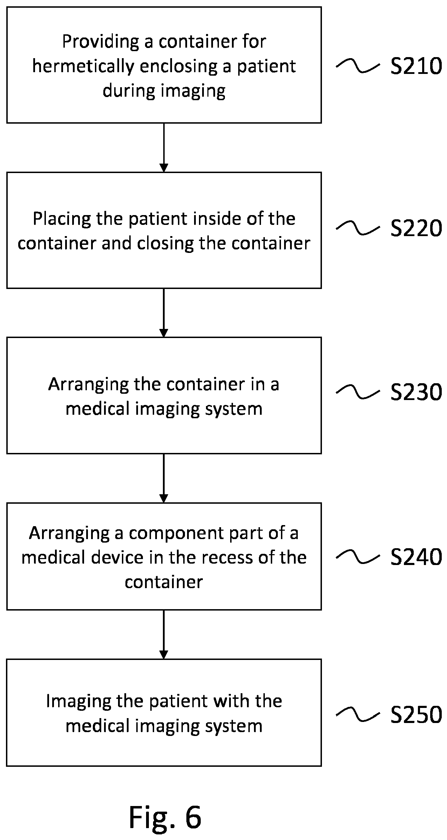

Fig. 6 shows a block diagram of a method of using a container 100 according to an embodiment of the invention in a medical imaging procedure. After providing a container 100 for hermetically enclosing a patient 110 during imaging in step S210, the patient 110 is placed inside of the container 100 and the container is closed in step S220. Then, in step S230, the container 100 with the patient 110 is arranged in a medical imaging system. A component part 150 of the medical imaging system like a body coil of a magnetic resonance imaging system is arranged in the recess 130 of the container 100 in step S240. In step S250, the patient 110 is imaged with the medical imaging system while being located inside of the diagnostic container 100.

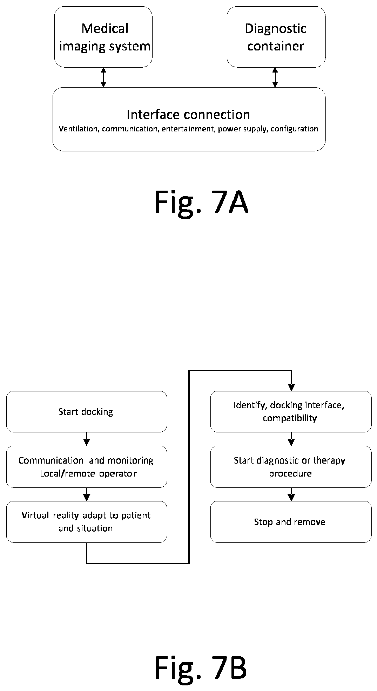

Fig. 7A shows a schematic set-up of an interface connection between a medical imaging system and a diagnostic container 100 according to an embodiment of the invention. The interface connection provides for ventilation of the patient 110, the supply of the container with electric power, communication of the container with the imaging system and configuration of the system, and may even provide entertainment features for comfort of the patient during the imaging procedure.

Fig. 7B shows a flowchart of a software protocol between a medical imaging system and a diagnostic container 100 according to an embodiment of the invention. A software protocol between the imaging system and the diagnostic container 100 controls the safe docking of the container and the workflow. A flowchart for the transport and docking of the container 100 to a diagnostic or therapy scanner is shown. After docking, the communication to a local or remote operator can be established and monitoring of the patient 110 is started. A virtual reality can be started that is adapted to the patient and its specific situation. The container 100 is identified by the system via the docking interface, and compatibility with the imaging system can be tested. Then the diagnostic or therapeutic procedure is started. After successfully finishing the procedure, the container can be disconnected and removed from the medical scanner, and the patient can be transported back inside the container 100.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: diagnostic container
- 110: patient
- 120: airtight shell
- 130: recess
- 140: inner volume
- *150*: component part
- 160: patient bed
- 170: access port
- 171: opening
- 172: seal
- 180: head coil
- 191: camera
- 192: light source
- 193: optical fiber
- 194: skin patch

## Claims

1. A diagnostic container (100) for hermetically enclosing a patient (110) during imaging with a medical imaging system; the container (100) comprising
an airtight shell (120) configured for providing an airtight enclosure of the patient (110);
wherein the airtight shell comprises at least one recess (130) extending into an inner volume (140) of the airtight shell, the recess (130) being configured for ensuring the airtight enclosure;
wherein the recess (130) is configured for receiving a component part (150) of a medical device and for allowing a positioning of the component part (150) in close proximity to the patient (110) without direct contact of the component part to the patient;
wherein the medical imaging system is a magnetic resonance imaging system and wherein the component part (150) of the medical device is a body coil of the magnetic resonance imaging system.

2. The container (100) according to claim 1, wherein the container comprises a patient bed (160) configured for receiving the patient (110), and wherein the patient bed (160) is arranged inside of the airtight shell.

3. The container (100) according to claim 1, wherein the container comprises a patient bed (160), wherein the airtight shell (120) is configured to be connected to the patient bed, and wherein the airtight enclosure of the patient (110) is provided by the airtight shell (120) in combination with the patient bed (160).

4. The container (100) according to any of the preceding claims, wherein the airtight shell (120) comprises an access port,
wherein the access port (170) comprises an opening (171) and a seal (172),
wherein the access port (170) is configured to allow insertion of a head coil (180) of the medical imaging system through the opening (171) to position the head coil (180) in close proximity to the head of the patient (110), and wherein the seal (172) is configured to ensure the airtight enclosure.

5. The container (100) according to any of the preceding claims, wherein the container comprises an interface configured to connect the container to the medical imaging system to provide electric power supply, ventilation and/or data transfer between the container (100) and the medical imaging system.

6. The container (100) according to any of the preceding claims, wherein the airtight shell (120) is a mechanical hardcover or a sliding tent roof.

7. The container (100) according to claim 5, wherein the interface is arranged at an end of the container corresponding with the head or with the feet of the patient (110).

8. The container (100) according to any of the preceding claims, wherein the container comprises an optical window configured for camera-based (191) monitoring of a vital sign of the patient (100) through the optical window.

9. The container (100) according to any of the preceding claims, wherein the container comprises a feedthrough for an optical fiber (193) configured to be attached to the body of the patient (110) via a skin patch (194).

10. The container (100) according to any of the preceding claims, wherein the airtight shell (120) is made of a material with low X-ray absorption and/or low absorption for radiation for oncology therapy treatment.

11. The container (100) according to any of the preceding claims, wherein the container comprises a marker configured to provide an indication of a position of the patient (110) with respect to the medical imaging system.

12. The container (100) according to any of the preceding claims, wherein the airtight shell (120) comprises a plurality of modules, wherein the modules are configured to be connected to each other in order to provide the airtight enclosure and/or wherein the modules comprise a communication link to be connected communicationally to each other.

13. The container (100) according to any of the preceding claims, wherein the container comprises an airlock with a membrane, wherein the airlock is configured to allow contact imaging of the body of the patient (110) through the membrane.

14. Use of a container (100) according to any of claims 1 to 13 in a medical imaging system.

## Patentansprüche

1. Diagnostischer Behälter (100) zum hermetischen Umschließen eines Patienten (110) während einer Bildgebung mit einem medizinischen Bildgebungssystem; der Behälter (100) umfassend
eine luftdichte Hülle (120), die so konfiguriert ist, dass sie den Patienten (110) luftdicht umschließt;
wobei die luftdichte Hülle mindestens eine Aussparung (130) umfasst, die sich in ein Innenvolumen (140) der luftdichten Hülle erstreckt, wobei die Aussparung (130) so konfiguriert ist, dass sie die luftdichte Umschließung gewährleistet;
wobei die Aussparung (130) so konfiguriert ist, dass sie ein Bauteil (150) einer medizinischen Vorrichtung aufnehmen kann und eine Positionierung des Bauteils (150) in unmittelbarer Nähe des Patienten (110) ermöglicht, ohne dass das Bauteil den Patienten direkt berührt;
wobei das medizinische Bildgebungssystem ein Magnetresonanztomographiesystem ist und wobei das Bauteil (150) der medizinischen Vorrichtung eine Körperspule des Magnetresonanztomographiesystems ist.

2. Behälter (100) nach Anspruch 1, wobei der Behälter ein Patientenbett (160) umfasst, das zum Aufnehmen des Patienten (110) konfiguriert ist, und wobei das Patientenbett (160) innerhalb der luftdichten Hülle angeordnet ist.

3. Behälter (100) nach Anspruch 1, wobei der Behälter ein Patientenbett (160) umfasst, wobei die luftdichte Hülle (120) so konfiguriert ist, dass sie mit dem Patientenbett verbunden werden kann, und wobei die luftdichte Umschließung des Patienten (110) durch die luftdichte Hülle (120) in Kombination mit dem Patientenbett (160) bereitgestellt wird.

4. Behälter (100) nach einem der vorstehenden Ansprüche, wobei die luftdichte Hülle (120) eine Zugangsöffnung umfasst,
wobei die Zugangsöffnung (170) eine Öffnung (171) und eine Dichtung (172) umfasst,
wobei die Zugangsöffnung (170) so konfiguriert ist, dass die Kopfspule (180) des medizinischen Bildgebungssystems durch die Öffnung (171) eingeführt werden kann, um die Kopfspule (180) in unmittelbarer Nähe des Kopfes des Patienten (110) zu positionieren, und wobei die Dichtung (172) so konfiguriert ist, dass die luftdichte Umschließung gewährleistet ist.

5. Behälter (100) nach einem der vorstehenden Ansprüche, wobei der Behälter eine Schnittstelle aufweist, die so konfiguriert ist, dass sie den Behälter mit dem medizinischen Bildgebungssystem verbindet, um eine Stromversorgung und Belüftung und/oder Datenübertragung zwischen dem Behälter (100) und dem medizinischen Bildgebungssystem bereitzustellen.

6. Behälter (100) nach einem der vorstehenden Ansprüche, wobei die luftdichte Hülle (120) eine mechanische Hartabdeckung oder ein verschiebbares Zeltdach ist.

7. Behälter (100) nach Anspruch 5, wobei die Schnittstelle an einem Ende des Behälters angeordnet ist, das dem Kopf oder den Füßen des Patienten (110) entspricht.

8. Behälter (100) nach einem der vorstehenden Ansprüche, wobei der Behälter ein optisches Fenster umfasst, das für die kamerabasierte (191) Überwachung eines Vitalzeichens des Patienten (100) durch das optische Fenster konfiguriert ist.

9. Behälter (100) nach einem der vorstehenden Ansprüche, wobei der Behälter eine Durchführung für eine optische Faser (193) umfasst, die so konfiguriert ist, dass sie über ein Hautpflaster (194) am Körper des Patienten (110) befestigt werden kann.

10. Behälter (100) nach einem der vorstehenden Ansprüche, wobei die luftdichte Hülle (120) aus einem Material mit geringer Röntgenabsorption und/oder geringer Absorption für Strahlung bei einer onkologischen Therapie gefertigt ist.

11. Behälter (100) nach einem der vorstehenden Ansprüche, wobei der Behälter eine Markierung umfasst, die so konfiguriert ist, dass sie eine Angabe über die Position des Patienten (110) in Bezug auf das medizinische Bildgebungssystem bereitstellt.

12. Behälter (100) nach einem der vorstehenden Ansprüche, wobei die luftdichte Hülle (120) mehrere Modulen umfasst, die so konfiguriert sind, dass sie miteinander verbunden werden können, um die luftdichte Umhüllung bereitzustellen, und/oder wobei die Module eine Kommunikationsverbindung umfassen, um miteinander kommunikativ verbunden zu werden.

13. Behälter (100) nach einem der vorstehenden Ansprüche, wobei der Behälter eine Schleuse mit einer Membran aufweist, wobei die Schleuse so konfiguriert ist, dass sie eine Kontaktbildgebung des Körpers des Patienten (110) durch die Membran ermöglicht.

14. Verwendung eines Behälters (100) nach einem der Ansprüche 1 bis 13 in einem medizinischen Bildgebungssystem.

## Revendications

1. Caisson de diagnostic (100) destiné à enfermer hermétiquement un patient (110) lors d'une imagerie avec un système d'imagerie médicale ; le caisson (100) comprenant
une coque étanche à l'air (120) configurée pour fournir une enceinte étanche à l'air au patient (110) ;
dans lequel la coque étanche à l'air comprend au moins un évidement (130) s'étendant dans un volume intérieur (140) de la coque étanche à l'air, l'évidement (130) étant configuré pour constituer l'enceinte étanche à l'air ;
dans lequel l'évidement (130) est configuré pour recevoir une partie constitutive (150) d'un dispositif médical et pour permettre un positionnement de la partie constitutive (150) à proximité immédiate du patient (110) sans contact direct de la partie constitutive avec le patient ;
dans lequel le système d'imagerie médicale est un système d'imagerie par résonance magnétique et dans lequel la partie constitutive (150) du dispositif médical est une bobine corps entier du système d'imagerie par résonance magnétique.

2. Caisson (100) selon la revendication 1, dans lequel le caisson comprend un lit de patient (160) configuré pour recevoir le patient (110), et dans lequel le lit de patient (160) est disposé à l'intérieur de la coque étanche à l'air.

3. Caisson (100) selon la revendication 1, dans lequel le caisson comprend un lit de patient (160), dans lequel la coque étanche à l'air (120) est configurée pour être connectée au lit de patient, et dans lequel l'enceinte étanche à l'air du patient (110) est composée de la coque étanche à l'air (120) en combinaison avec le lit de patient (160).

4. Caisson (100) selon l'une quelconque des revendications précédentes, dans lequel la coque étanche à l'air (120) comprend un orifice d'accès,
dans lequel l'orifice d'accès (170) comprend une ouverture (171) et un joint (172),
dans lequel l'orifice d'accès (170) est configuré pour permettre l'insertion d'une bobine de tête (180) du système d'imagerie médicale à travers l'ouverture (171) afin de positionner la bobine de tête (180) à proximité immédiate de la tête du patient (110), et dans lequel le joint (172) est configuré pour consituer l'enceinte étanche à l'air.

5. Caisson (100) selon l'une quelconque des revendications précédentes, dans lequel le caisson comprend une interface configurée pour connecter le caisson au système d'imagerie médicale afin de fournir une alimentation électrique, une ventilation et/ou un transfert de données entre le caisson (100) et le système d'imagerie médicale.

6. Caisson (100) selon l'une quelconque des revendications précédentes, dans lequel la coque étanche à l'air (120) est un couvercle rigide mécanique ou un toit en toile coulissant.

7. Caisson (100) selon la revendication 5, dans lequel l'interface est disposée à une extrémité du caisson correspondant à la tête ou aux pieds du patient (110).

8. Caisson (100) selon l'une quelconque des revendications précédentes, dans lequel le caisson comprend une fenêtre optique configurée pour la surveillance par caméra (191) d'un signe vital du patient (100) à travers la fenêtre optique.

9. Caisson (100) selon l'une quelconque des revendications précédentes, dans lequel le caisson comprend un passage pour une fibre optique (193) configurée pour être fixée au corps du patient (110) par l'intermédiaire d'un timbre cutané (194).

10. Caisson (100) selon l'une quelconque des revendications précédentes, dans lequel la coque étanche à l'air (120) est constituée d'un matériau à faible absorption des rayons X et/ou à faible absorption des radiations pour les traitements de radiothérapie oncologique.

11. Caisson (100) selon l'une quelconque des revendications précédentes, dans lequel le caisson comprend un marqueur configuré pour fournir une indication d'une position du patient (110) par rapport au système d'imagerie médicale.

12. Caisson (100) selon l'une quelconque des revendications précédentes, dans lequel la coque étanche à l'air (120) comprend une pluralité de modules, dans lequel les modules sont configurés pour être connectés entre eux afin de constituer l'enceinte étanche à l'air et/ou dans lequel les modules comprennent une liaison de communication permettant de les connecter en communication entre eux.

13. Caisson (100) selon l'une quelconque des revendications précédentes, dans lequel le caisson comprend un sas avec une membrane, dans lequel le sas est configuré pour permettre l'imagerie par contact du corps du patient (110) à travers la membrane.

14. Utilisation d'un caisson (100) selon l'une quelconque des revendications 1 à 13 dans un système d'imagerie médicale.
